(19) Europäisches Patentamt
European Patent Office
Office européen des brevets

(11) Veröffentlichungsnummer: **0 187 620 B1**

# (12) EUROPÄISCHE PATENTSCHRIFT

(45) Veröffentlichungstag der Patentschrift: **17.04.91**

(51) Int. Cl.[5]: **C07D 487/04**, G03G 5/06, G03G 5/05, //(C07D487/04, 209:00,209:00)

(21) Anmeldenummer: **85810611.5**

(22) Anmeldetag: **23.12.85**

## (54) Dithioketo-pyrrolo-pyrrole, Verfahren zu deren Herstellung und Verwendung.

(30) Priorität: **03.01.85 CH 14/85**

(43) Veröffentlichungstag der Anmeldung:
**16.07.86 Patentblatt 86/29**

(45) Bekanntmachung des Hinweises auf die Patenterteilung:
**17.04.91 Patentblatt 91/16**

(84) Benannte Vertragsstaaten:
**CH DE FR GB IT LI**

(56) Entgegenhaltungen:
**EP-A- 0 061 426**
**EP-A- 0 133 156**
**DE-B- 2 822 113**
**EP-A- 0 094 911**
**DD-A- 220 028**
**FR-A- 1 540 722**

(73) Patentinhaber: **CIBA-GEIGY AG**
**Klybeckstrasse 141**
**CH-4002 Basel(CH)**

(72) Erfinder: **Rochat, Alain Claude, Dr.**
**Route de Schiffenen 38**
**CH-1700 Fribourg(CH)**
Erfinder: **Iqbal, Abul, Dr.**
**Im Schaiengarten 1**
**CH-4107 Ettingen(CH)**
Erfinder: **Jeanneret, Rémy, Dr.**
**Feldstr. 110**
**CH-4123 Allschwil(CH)**
Erfinder: **Mizuguchi, Jin, Dr.**
**Route du Centre 17**
**CH-1723 Marly(CH)**

EP 0 187 620 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Dithioketo-pyrrolo-pyrrole sowie ihre Verwendung als Pigmente und insbesondere als photoleitfähige Substanzen.

Diketo-pyrrolo-pyrrole und ihre Eignung als Pigmente sind z.B. aus EP-A-0 061 426 bekannt. Es wird auch offenbart, dass sie als photoelektrophoretische Toner Verwendung finden können. Es wurden nun Dithioketo-pyrrolo-pyrrole gefunden, die sich ausgezeichnet als photoleitfähige Substanzen eignen.

Die Erfindung betrifft neue Pyrrolo-pyrrole der Formel (1)

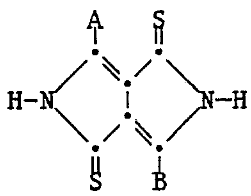

(1),

worin A und B gleiche oder verschiedene Reste der Formel (2)

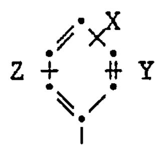

(2)

bedeuten, worin X, Y und Z H- oder Halogenatome, Trifluormethyl-, Cyangruppen, Alkyl-, Alkoxy- oder Alkylmercaptogruppen mit 1-12 C-Atomen, Alkoxycarbonyl- oder Dialkylaminogruppen mit 2-6 C-Atomen, gegebenenfalls durch Halogen, Alkyl oder Alkoxy mit 1-6 C-Atomen substituierte Phenoxy-, Phenylmercapto- oder Phenoxycarbonylgruppen bedeuten und mindestens einer der Substituenten X, Y und Z ein H-Atom bedeutet oder A und B Diphenyl oder Naphthyl bedeuten.

Die Substituenten X, Y und Z befinden sich beispielsweise in ortho-, meta- oder para-, vorzugsweise aber in meta-oder para-Stellung zur Dithioketo-pyrrolo-pyrrol-Gruppe.

Besonders bevorzugt sind jene Verbindungen der Formel (1), worin A und B gleiche oder verschiedene Reste der Formel (3)

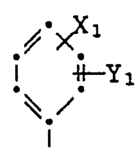

(3)

bedeuten, worin einer der Substituenten $X_1$ und $Y_1$ ein H-, Chlor-oder Bromatom, eine Methyl-, Cyan-, N,N-Dimethylamino- oder N,N-Diäthylaminogruppe, eine Alkoxygruppe mit 1-12 C-Atomen, eine Alkylmercaptogruppe mit 1-12 C-Atomen oder eine Alkoxycarbonylgruppe mit 2-4 C-Atomen und der andere ein H-Atom bedeuten. $X_1$ und $Y_1$ befinden sich beispielsweise in ortho-, meta- oder para-, vorzugsweise aber in meta- oder para-Stellung zur Dithioketo-pyrrolo-pyrrol-Gruppe.

Zu den Verbindungen der Formel (1) gelangt man beispielsweise, wenn man ein Diketo-pyrrolo-pyrrol der Formel (4)

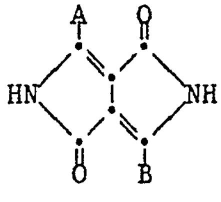

(4),

worin A und B die oben angegebene Bedeutung haben, mit einem Thionierungsmittel erhitzt. Es eignen

sich die bekannten Thionierungsmittel wie Tetraphosphortrisulfid ($P_4S_3$), Tetraphosphorheptasulfid ($P_4S_7$), Tetraphosphordekasulfid ($P_4S_{10}$) sowie dessen Pyridinkomplex $P_4S_{10}\cdot 4C_5H_5N$, ferner Dithiaphosphetane und insbesondere das 2,4-Bis-(4-methoxyphenyl)-1,3-dithia-2,4-diphosphetan-2,4-disulfid (Lawesson's Reagenz). Letzteres und Tetraphosphordekasulfid sind bevorzugt. Die Umsetzung wird zweckmässig bei Temperaturen zwischen 50-250° mit einem Ueberschuss des Thionierungsmittels, vorzugsweise zwischen 80 und 150°C in einem inerten organischen Lösungsmittel durchgeführt. Als Lösungsmittel eignen sich beispielsweise aromatische Kohlenwasserstoffe, wie Benzol, Toluol, Xylol oder Tetrahydronaphthalin, chlorierte aromatische Kohlenwasserstoffe, wie Chlorbenzol und O-Dichlorbenzol, Aether, wie Dimethoxyäther, Diäthylenglykoldimethyläther, Anisol, Dioxan oder Diphenyläther oder auch Nitrile, wie Acetonitril, Benzonitril oder Amide bzw. Thioamide, wie z.B. Dimethylthioformamid, Dimethylthioacetamid, Tetraäthylsulfamid und Hexamethylphosphorsäuretriamid oder Gemische der erwähnten Lösungsmittel.

Die erhaltenen Dithioketo-pyrrolo-pyrrole können in der Regel durch Abfiltrieren isoliert werden. Je nach Verwendung ist eine Nachbehandlung erforderlich, um die chemische Reinheit zu erhöhen (z.B. Rekristallisation, Sublimation, usw.) und/oder um die Kristallform zu verändern (z.B. Konditionierung in einem organischen Lösungsmittel). Dazu erweisen sich folgende Lösungsmittel als besonders geeignet: Durch Halogenatome, Alkyl- oder Nitrogruppen substituierte Benzole, wie Xylole, Chlorbenzol, o-Dichlorbenzol oder Nitrobenzol sowie Pyridinbasen, wie Pyridin, Picolin oder Chinolin, ferner Ketone, wie Aceton, Methyläthylketon oder Cyclohexanon, Aether, wie Aethylenglykolmonomethyl- oder monoäthyläther oder Tetrahydrofuran, Amide, wie Dimethylformamid oder N-Methyl-pyrrolidon, Alkohole, wie Methanol oder Aethanol und ferner Dimethylsulfoxyd, Sulfolan, Acetonitril, Benzonitril und Methyl- sowie Aethylacetat. Bevorzugt werden Aceton, Tetrahydrofuran, Dimethylformamid, Methanol, Dimethylsulfoxid, Aethylacetat oder Acetonitril.

Je nach Art der Reste A und B und des zu färbenden Polymeren können die Verbindungen der Formel (1) als polymerlösliche Farbstoffe oder als Pigmente verwendet werden.

Von besonderem Interesse sind die Verbindungen der Formel (1) als photoleitfähige Substanzen, beispielsweise in elektrophotographischen Aufzeichnungsmaterialien. Solche bestehen aus einer leitfähigen Unterlage und einem Schichtaufbau, der im Dunkeln isolierend ist, aber unter Belichtung leitend wird. Der Aufbau kann aus einer oder mehreren Schichten bestehen. Im Fall einer einzigen Schicht ist mindestens eine photoleitfähige Substanz in mindestens einem Bindemittel dispergiert oder unmittelbar auf eine leitende Unterlage aufgedampft. Ein mehrschichtiger Aufbau besteht aus mindestens einer ladungsträgererzeugenden Schicht, enthaltend eine oder mehrere photoleitfähige Substanzen, und mindestens einer ladungstransportierenden Schicht.

Eine weitere Ausführungsform der vorliegenden Erfindung ist demnach ein elektrophotographisches Aufzeichnungsmaterial, bestehend aus mindestens einer leitfähigen Unterlage, einer bei Belichtung ladungserzeugenden Schicht und einer ladungstransportierenden Schicht, wobei in mindestens einer dieser Schichten mindestens eine Verbindung der Formel (1) enthalten ist.

Die leitfähige Unterlage kann aus einer Metallplatte oder -folie bestehen, die roh oder z.B durch Aufrauhen vorbehandelt ist und z.B. aus Aluminium, Zink, Magnesium, Kupfer oder einer Legierung dieser Metalle besteht. Im Falle des Aluminiums kann die Vorbehandlung in einem Anodisieren bestehen. Als Unterlagen kommen auch Aluminiumbedampfte Kunststofffolien sowie Polymerfilme mit metallisierter Oberfläche in Frage.

Der Schichtaufbau enthält die Dithioketo-pyrrolo-pyrrole als bei Belichtung ladungserzeugende Substanzen, welche mit im Aufbau vorhandenen ladungstransportierenden Substanzen zusammenwirken. Ein solcher Aufbau erlaubt nach vorgängiger statischer Aufladung und bildmässiger Belichtung die Erzeugung eines entsprechenden Musters aufgeladener und entladener Stellen (latentes Bild), welches nach bekannten Verfahren der Reprographie in ein sichtbares Abbild übergeführt werden kann.

Die Belichtung kann mit Licht im sichtbaren Wellenbereich erfolgen. Es ist aber ein besonderer Vorzug der Dithioketo-pyrrolo-pyrrole, dass sie auch Strahlung im nahen Infrarot-Bereich zu absorbieren vermögen und dass sie in diesem Wellenlängenbereich auch Photoleitung zeigen. Von besonderem Interesse ist dabei der Bereich 700-900 nm, in welchem die energieintensiven Galliumarsenid-Laser arbeiten.

Zur Erhaltung des statischen Potentials an Stellen, die nicht belichtet werden, tragen die Dithioketo-pyrrolo-pyrrole dadurch bei, dass sie einen hohen Dunkelwiderstand aufweisen.

Besteht der Schichtaufbau aus einer einzigen Schicht, so enthält diese ein oder mehrere Dithioketo-pyrrolo-pyrrole in fein verteilter Form, gegebenenfalls zusammen mit ladungstransportierenden Substanzen in einem organischen Bindemittel. Das Bindemittel ist filmbildend, isolierend und adhäsiv. Je nach Anwendung ist es löslich in organischen Lösungsmitteln oder in basischen Mischungen organischer Lösungsmittel, die gegebenenfalls Wasser enthalten. Besonders geeignet sind Bindemittel auf der Basis von Polykondensations-und Polyadditionsprodukten wie Polyamiden, Polyurethanen, Polyestern, Epoxyharzen, Phenoxyharzen, Polyketonen, Polycarbonaten, Polyvinylketonen, Polystyrolen, Polyvinylcarbazolen, Polyacrylamiden, Polymethylmethacrylaten, Polyvinylbutyraten, Polyvinylchloriden sowie Copolymerisaten wie z.B. Styrol-Maleinsäureanhydrid-Copolymeren oder Styrol-Methacrylsäure-Methacrylsäureester-Copolymeren.

Besteht der Schichtaufbau aus mehreren Schichten, so steht die Doppelschicht im Zentrum des Interesses. Für diesen Fall wird auf die leitfähige Unterlage zuerst eine ladungserzeugende und auf diese eine zweite ladungstransportierende Schicht aufgebracht. Das Aufbringen der Schichten kann auch in umgekehrter Reihenfolge durchgeführt werden. Eine der Schichten, vorzugsweise die ladungserzeugende Schicht enthält mindestens ein Dithioketo-pyrrolo-pyrrol. Dieses kann in einem organischen Bindemittel gelöst oder fein verteilt sein. Die Applikation auf die leitfähige Unterlage erfolgt beispielsweise durch Auftragen einer Lösung beziehungsweise Dispersion der Bindemittel-Farbkörper-Mischung in einem organischen Lösungsmittel und anschliessendes Verdampfen des Lösungsmittels. Man kann die Dithioketo-pyrrolo-pyrrole aber auch auf die leitfähige Unterlage aufdampfen.

Die zweite Schicht enthält eine oder mehrere ladungstransportierende Substanzen, vorzugsweise gelöst oder dispergiert in einem organischen Bindemittel. Als ladungstransportierende Substanzen kommen die verschiedensten aromatischen, vorzugsweise stickstoffhaltigen Verbindungen in Frage wie Hydrazone oder aromatische Amine, die gegebenenfalls Alkylidenbrücken oder -reste enthalten. Beispielsweise handelt es sich um die in der Deutschen Offenlegungsschrift 34 47 685 auf den Seiten 57-65 beschriebenen Substanzen.

Obwohl die erfindungsgemässen Pigmente eine Endabsorption bis 780 nm aufweisen, sind sie als photoleitende Substanzen für die Laser-Aufzeichnung noch ungenügend. Es wurde nun allerdings eine Methode gefunden, die es erlaubt diese Absorption in entscheidendem Masse nach höheren Wellenlängen zu verschieben. Dies geschieht durch Aussetzen des wie oben beschriebenen erhaltenen photographischen Aufzeichnungsmaterials einem Lösungsmitteldampf, wie z.B. Aceton-, Tetrahydrofuran-, Dimethylformamid-, Methanol-, Acetonitril-, Dimethylsulfoxid- oder Aethylacetatdampf während 1-2 Stunden.

Aus der Publikation von K. Arishima et al., Appl. Phys. Letters 40 (3), S. 279 (1982), ist diese Methode zur Verschiebung der Wellenlänge für bestimmte Phthalocyanine bekennt. Man erreicht dabei eine Verschiebung nach längeren Wellenlängen von ca. 90 nm. Es wurde deshalb versucht dieselbe Methode auf 1,4-Diketopyrrolo-[3,4-c]-pyrrole anzuwenden, man erhielt jedoch eine Verschiebung nach kürzeren Wellenlängen. Ueberraschenderweise ergibt die Anwendung dieser Methode auf Materialien, welche die erfindungsgemässen Dithioketo-pyrrolo-pyrrole enthalten eine ganz unerwartete Verschiebung nach längeren Wellenlängen von ca. 130 und mehr nm. Diese Lösungsmittelbehandlung zeigt ebenfalls eine Erhöhung des Dunkel-Widerstandes und der Photoleitfähigkeit um ca. 100 Einheiten.

Dieselbe spektrale Verschiebung, sowie ähnliche Werte für den Dunkel-Widerstand und den Photostrom werden auch mit den gepulverten Pigmenten erhalten; diese Pigmente werden zuerst gemahlen, dann mit einem Lösungsmittel, wie z.B. Aethylacetat behandelt und anschliessend mit Hilfe eines Bindemittels auf eine Unterlage aufgetragen.

Eine weitere Ausführungsform der Erfindung betrifft demnach die Herstellung eines elektrophotographischen Aufzeichnungsmaterials, dadurch gekennzeichnet, dass man eine Verbindung der Formel (1) auf eine leitende Unterlage mit Bindemitteln aufträgt oder im Vakuum aufdampft, die so hergestellte Schicht mit einem organischen Lösungsmittel im flüssigen oder gasförmigen Zustand behandelt und anschliessend eine zweite Schicht, enthaltend eine aromatische, stickstoffhaltige Verbindung, aufbaut.

Die ladungserzeugende und ladungstransportierende Schicht kann noch Zusätze, wie Egalisiermittel, oberflächenaktive Mittel oder Weichmacher enthalten, um die physikalischen Eigenschaften der Schichten zu verbessern.

Die nachstehenden Beispiele erläutern die Erfindung:

Beispiel 1 : In einem 200 ml Sulfierkolben mit Propellerrührer, Thermometer und Rückflusskühler, werden unter Stickstoff-Atmosphäre 2,89 g 1,4-Diketo-3,6-diphenyl-pyrrolo-[3,4-c]-pyrrol und 4,95 g 2, 4-Bis(4-methoxyphenyl)-1, 3-dithia-2, 4-diphospetan-2,4-disulfid (Lawesson's Reagenz) vorgelegt und mit 60 ml Xylolgemisch und 2,5 ml Hexamethyl-phosphorsäuretriamid überdeckt. Das Gemisch wird kräftig verrührt und am Rückfluss bei 133 °C erwärmt. Nach 7-stündigem Kochen kühlt man es auf ca. 90 °C, filtriert und wäscht das entstandene Produkt mit warmem Xylol, dann mit wenig Aceton und schliesslich mit Methanol und trocknet es bei 90 °C unter Vakuum. Man erhält 3,04 g der Verbindung der Formel

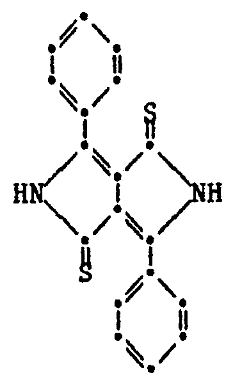

als schwarzblaues, kristallines Pulver.

| | | | |
|---|---|---|---|
| Analyse: | C = 65,73 % | N = 7,88 % | Schmelzpunkt über 350°C unter Zersetzung |
| | H = 4,00 % | S = 17,2 % | |

Nach MS-, NMR- sowie Elementaranalyse entspricht dies folgender Summenformel $C_{18}H_{12}N_2S_2 \cdot 1/2\ H_2O$ (Mol = 320,4; + 1/2 $H_2O$)

Zur Erhaltung eines besonders reinen Produkts kann das Rohprodukt am besten aus Dimethylsulfoxid umkristallisiert und anschliessend, nach Filtration, in Methanol nach üblicher Methode konditioniert werden.

Beispiel 2 : In einem 200 ml Sulfierkolben mit Propellerrührer, Thermometer und Rückflusskühler, werden unter Stickstoff-Atmosphäre 2,21 g 1,4-Diketo-3,6-di(p-tolyl)-pyrrolo-[3,4-c]-pyrrol und 3,47 g Lawesson's Reagenz vorgelegt und mit 70 ml Xylolgemisch und 2,0 ml Hexamethyl-phosphorsäuretriamid überdeckt. Das Gemisch wird kräftig verrührt und am Rückfluss bei 132° C erwärmt. Da es wesentlich dicker wird, muss man es mit 10 ml Xylolgemisch verdünnen. Nach 12-stündigem Kochen am Rückfluss kühlt man es auf ca. 80° C, filtriert das entstandene Produkt und wäscht es nacheinander mit Xylolgemisch und Methanol, und trocknet es bei 100° C im Vakuum. Man erhält 1,47 g der Verbindung der Formel

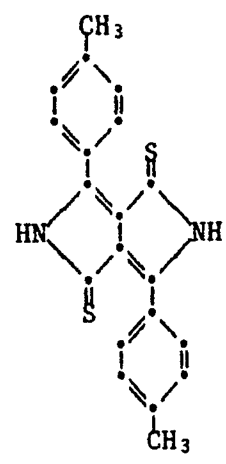

als dunkelblaues Pulver.

| | | | |
|---|---|---|---|
| Analyse: | C = 68,06 % | N = 7,71 % | Schmelzpunkt über 350°C unter Zersetzung |
| | H = 4,76 % | S = 16,86 % | |

Nach MS-, NMR- sowie Elementaranalyse entspricht dieses neue Rohprodukt folgender Summenformel: $C_{20}H_{16}N_2S_2$ mit wenig Wasser (Mol 348,3 + wenig Wasser).

Beispiel 3 : 1,74 g 1,4-Diketo-3,6-di-(4-methoxyphenyl)-pyrrolo-[3,4-c]-pyrrol und 2,48 g Lawesson's Reagenz werden genau nach der Arbeitsweise von Beispiel 2 umgesetzt. Keine zusätzliche Verdünnung ist dabei nötig. Man erhält nach der Trocknung 1,83 g der Verbindung der Formel

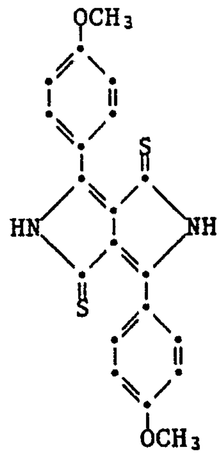

als dunkelblaues Pulver.

| | | | |
|---|---|---|---|
| Analyse: | C = 62,9 % | N = 7,25 %: | Schmelzpunkt über 350°C unter Zersetzung |
| | H = 4,35 % | S = 16,2 % | |

Die Struktur des Pigmentes wird durch spektroskopische Daten (IR, NMR und MS) bestätigt.

Beispiel 4 : In einem 100 ml Sulfierkolben mit Propellerrührer, Thermometer und Rückflusskühler werden unter Stickstoff 1,38 g 1,4-Diketo-3,6-bis(4-dodecylmerkaptophenyl)-pyrrolo[3,4-c]pyrrol, 0,99 g Lawesson's Reagenz und 30 ml Xylolgemisch vorgelegt. Das Gemisch wird auf 130° C erwärmt und bei dieser Temperatur während 2 Std. gehalten. Anschliessend kühlt man auf 80° C ab, filtriert das Produkt und wäscht es nacheinander mit heissem Xylolgemisch und mit Methanol. Die Trocknung im Vakuumofen bei ca. 100° C liefert 0,400 g der Verbindung der Formel

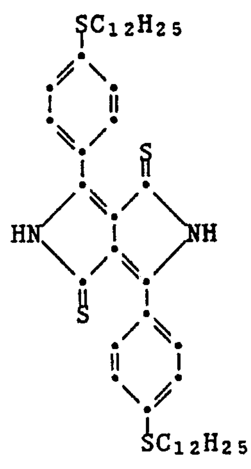

als dunkelblaues Pulver. Smp. 287-289° C (Zers.).

Analyse: C = 69,3 %, H = 8,33 %, N = 3,82 %, S = 17,8 %.

Beispiel 5 : 0,54 g des gemäss Beispiel 1 erhaltenen Pigments werden in 14,4 g einer 7,5 gew.-%igen Lösung von Lucite® 41 (einem Polymethylmethacrylat, hergestellt von DuPont) in Methyläthylketon mit 70 g Glaskugeln von 4-5 mm Durchmesser in einer Flasche von 50 ml Inhalt während 16 Std. auf einer Vibrationskugelmühle (Typ Vibratom® der Firma Siebtechnik in Mühlheim/Ruhr) dispergiert. Nach Abtrennen der Glaskugeln wird die Pigmentdispersion mit einem Ziehstab von nominell 150 µm Nassfilmdicke auf eine Aluminiumunterlage aufgestrichen. Nach der Trocknung resultiert eine Schicht mit einer Dicke von ca. 15 µm, welche elektrophotographische Eigenschaften zeigt (E 1/2 ca. 10 µJ/cm²).

Beispiel 6 : Es wird gleich verfahren wie in Beispiel 5, nur dass an Stelle des Pigments von Beispiel 1 das Pigment von Beispiel 4 eingesetzt wird. Es resultiert eine Schicht mit guten elektrophotographischen

Eigenschaften.

Beispiel 7 : 0,3 g des Pigments vom Beispiel 1 werden in einem Gemisch von 10 g Aethanol und Methyläthylketon (2:1 in Volumen), enthaltend 0,2 g Aethylcellulose, aufgenommen. Die Suspension wird dann während 5 Std. mit Glaskugeln gemahlen und anschliessend auf eine Aluminiumplatte mit einem Ziehstab aufgetragen (= ladungserzeugende Schicht). Diese Schicht wird bei 50°C während 3 Std. getrocknet. Die Schichtdicke beträgt 6 µm. Eine zweite Schicht, bestehend aus einem Gemisch von 0,6 g eines Hydrazons der Formel (5)

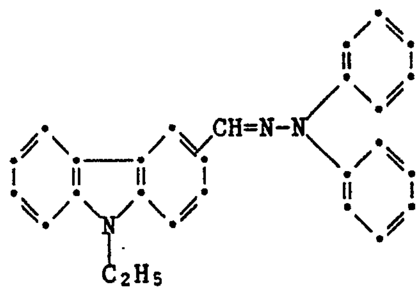

(5),

wie es z.B. in der DE-OS 2 919 791 beschrieben ist, und 0,9 g Lucite® 41 in 11 g Methyläthylketon, wird dann aufgetragen und bei 50°C während 15 Std. getrocknet. Die Schichtdicke beträgt 10 bis 15 µm. Dieses Photorezeptor zeigt eine Empfindlichkeit (E 1/2) von 5 µJ/cm² und die Aufladbarkeit beträgt 430 Volts.

Beispiel 8 : Das Pigment vom Beispiel 1 wird mit einer Geschwindigkeit von 5 A/Sek unter einem Vakuum von $10^{-6}$ mbar auf eine Aluminium-Unterlage aufgedampft. Die erhaltene Schichtdicke beträgt 2000 bis 3000 A. Dieser Film wird bei Raumtemperatur während 1 Std. dem Dampf von Methanol ausgesetzt. Eine zweite Schicht von derselben Zusammensetzung wie im Beispiel 7 wird anschliessend aufgetragen. Der Film zeigt eine Absorption bei 830 nm.

Beispiel 9 : Das Pigment vom Beispiel 4 wird wie im Beispiel 8 aufgedampft und der erhaltene Film einem Dampf von Aceton während 1 Std. ausgesetzt. Eine zweite Schicht wird darüber aufgetragen, deren Zusammensetzung derjenigen vom Beispiel 7 entspricht. Der Film zeigt eine Absorption bei 830 nm.

Beispiel 10 : 0,4 g des Pigmentes vom Beispiel 1 werden in 10 ml destilliertem Wasser mit 40 g Glaskugeln von 1 mm Durchmesser während 2 Tagen gemahlen. Das erhaltene Produkt wird filtriert und bei 50°C während 24 Std. getrocknet, anschliessend in Aethylacetat während 1 Std. getränkt, und wieder filtriert und getrocknet. Die Herstellung der Monoschicht erfolgt wie im Beispiel 5. Der Film zeigt eine Absorption bei 830 nm.

Beispiel 11 : Mit dem Pigment vom Beispiel 1, welches wie im Beispiel 10 vorbehandelt wird, erzeugt man eine Doppel-Schicht nach der Vorschrift von Beispiel 7.

Beispiel 12 : Das Pigment vom Beispiel 4 wird wie im Beispiel 10 vorbehandelt und damit eine Doppelschicht nach der Vorschrift von Beispiel 7 hergestellt.

## Ansprüche

1. Verbindungen der Formel (1)

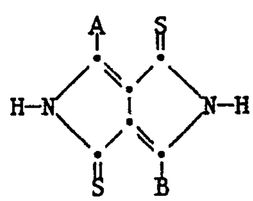

(1),

worin A und B gleiche oder verschiedene Reste der Formel (2)

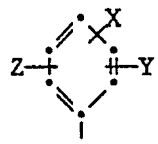

(2)

bedeuten, worin X, Y und Z H- oder Halogenatome, Trifluormethyl-, Cyangruppen, Alkyl-, Alkoxy- oder Alkylmercaptogruppen mit 1-12 C-Atomen, Alkoxycarbonyl- oder Dialkylaminogruppen mit 2-6 C-Atomen, gegebenenfalls durch Halogen, Alkyl oder Alkoxy mit 1-6 C-Atomen substituierte Phenoxy-, Phenylmercapto-, Phenoxycarbonylgruppen bedeuten und mindestens einer der Substituenten X, Y und Z ein H-Atom bedeutet oder A und B Diphenyl oder Naphthyl bedeuten.

**2.** Verbindungen gemäss Anspruch 1, worin A und B gleiche oder verschiedene Reste der Formel (3)

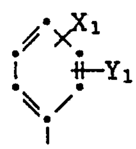

(3)

bedeuten, worin einer der Substituenten $X_1$ und $Y_1$ ein H-, Chlor- oder Bromatom, eine Methyl-, Cyan-, N,N-Dimethylamino- oder N,N-Diäthylaminogruppe, eine Alkoxygruppe mit 1-12 C-Atomen oder eine Alkylmercaptogruppe mit 1-12 C-Atomen, eine Alkoxycarbonylgruppe mit 2-4 C-Atomen und der andere ein H-Atom bedeuten.

**3.** Verbindungen gemäss Ansprüchen 1 und 2, worin sich die Substituenten X, Y und Z bzw. $X_1$ und $Y_1$ in meta- oder p-Stellung zur Dithioketo-pyrrolo-pyrrolgruppe befinden.

**4.** Verfahren zur Herstellung der Verbindungen der Formel (1) gemäss Anspruch 1, dadurch gekennzeichnet, dass man ein Diketo-pyrrolo-pyrrol der Formel (4)

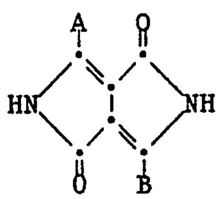

(4),

worin A und B die in Anspruch 1 angegebene Bedeutung haben, mit einem Thionierungsmittel erhitzt.

**5.** Verfahren gemäss Anspruch 4, dadurch gekennzeichnet, dass man als Thionierungsmittel 2,4-Bis-(4-methoxyphenyl)-1,3-dithia-2,4-diphosphetan-2,4-disulfid oder Tetraphosphordekasulfid verwendet.

**6.** Verfahren gemäss Anspruch 4, dadurch gekennzeichnet, dass man die Thionierung in einem inerten organischen Lösungsmittel durchführt.

**7.** Verwendung der Verbindungen gemäss Anspruch 1 als photoleitfähige Substanzen.

**8.** Elektrophotographisches Aufzeichnungsmaterial, bestehend mindestens aus einer leitfähigen Unterlage, einer bei Belichtung ladungserzeugenden Schicht und einer ladungstransportierenden Schicht, dadurch gekennzeichnet, dass in mindestens einer dieser Schichten mindestens eine Verbindung der Formel (1) gemäss Anspruch 1 enthalten ist.

**9.** Elektrophotographisches Aufzeichnungsmaterial gemäss Anspruch 8, dadurch gekennzeichnet, dass die ladungserzeugende Schicht eine Verbindung gemäss Anspruch 2 enthält.

**10.** Herstellung eines elektrophotographischen Aufzeichnungsmaterials, dadurch gekennzeichnet, dass man

eine Verbindung gemäss Anspruch 1 auf eine leitende Unterlage mit Bindemittel aufträgt oder im Vakuum aufdampft, die so hergestellte Schicht mit einem organischen Lösungsmittel in flüssiger oder gasförmiger Form behandelt und anschliessend eine 2. Schicht, enthaltend eine aromatische, stickstoffhaltige Verbindung, aufbaut.

**11.** Herstellung eines elektrophotographischen Aufzeichnungsmaterials gemäss Anspruch 10, dadurch gekennzeichnet, dass man ein Lösungsmittel, ausgewählt aus der Gruppe bestehend aus Aceton, Tetrahydrofuran, Dimethylformamid, Methanol, Dimethylsulfoxid, Aethylacetat und Acetonitril verwendet.

## Claims

**1.** A compound of formula (1)

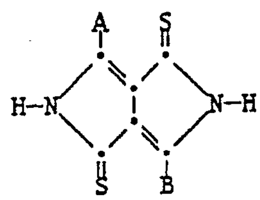

(1)

in which A and B are identical or different radicals of formula (2)

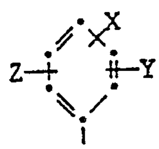

(2)

in which X, Y and Z are hydrogen or halogen atoms, trifluoromethyl or cyano groups, alkyl, alkoxy or alkylmercapto groups having 1 to 12 carbon atoms, alkoxycarbonyl or dialkylamino groups having 2 to 6 carbon atoms, phenoxy, phenylmercapto or phenoxycarbonyl groups each unsubstituted or substituted by halogen, alkyl or alkoxy having 1 to 6 carbon atoms, and in which at least one of the substituents X, Y and Z is a hydrogen atom or A and B are diphenyl or naphthyl.

**2.** A compound according to Claim 1, in which A and B are identical or different radicals of formula (3)

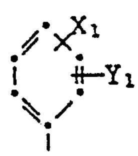

(3)

in which one of the substituents $X_1$ and $Y_1$ is a hydrogen, chlorine or bromine atom, a methyl, cyano, N,N-dimethylamino or N,N-diethylamino group, an alkoxy group having 1 to 12 carbon atoms or an alkylmercapto group having 1 to 12 carbon atoms, an alkoxycarbonyl group having 2 to 4 carbon atoms and the other is a hydrogen atom.

**3.** A compound according to Claims 1 and 2, in which the substituents X, Y and Z or $X_1$ and $Y_1$ are in the meta- or p-position to the dithioketopyrrolopyrrole group.

**4.** A process for the preparation of a compound of formula (1) according to Claim 1, which process comprises heating a diketopyrrolopyrrole of formula (4)

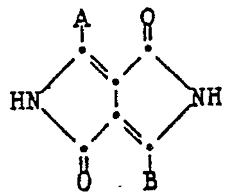

(4)

in which A and B are as defined in Claim 1, with a thionating agent.

5. A process according to Claim 4, wherein the thionating agent used is 2,4-bis-(4-methoxyphenyl)-1,3-dithia-2,4-diphosphetane 2,4-disulfide or tetraphosphorus decasulfide.

6. A process according to Claim 4, wherein the thionation is carried out in an inert organic solvent.

7. Use of a compound according to Claim 1 as photoconductive substance.

8. Electrophotographic recording material consisting of at least one conductive support, one layer which when subjected to exposure generates charges and one charge-transporting layer, at least one of which layers contains at least one compound of formula (1) according to Claim 1.

9. Electrophotographic recording material according to Claim 8, in which the layer which generates charges contains a compound according to Claim 2.

10. Preparation of an electrophotographic recording material, which comprises applying a compound according to Claim 1 with binders to a conductive support or vaporizing said compound onto said support in vacuo, treating the layer so prepared with an organic solvent in the liquid or gaseous state and subsequently adding a second layer containing an aromatic, nitrogen-containing compound.

11. Preparation of an electrophotographic recording material according to Claim 10, wherein the solvent used is selected from the group consisting of acetone, tetrahydrofuran, dimethylformamide, methanol, dimethyl sulfoxide, ethyl acetate and acetonitrile.

## Revendications

1. Composés répondant à la formule 1 :

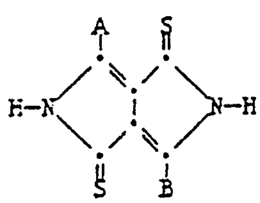

(1).

dans laquelle :

A et B représentent chacun, indépendamment l'un de l'autre, un radical répondant à la formule 2 :

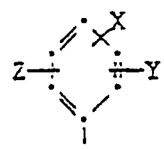

(2)

dans laquelle X, Y et Z représentent chacun un atome d'hydrogène ou d'halogène, un trifluorométhyle, un cyano, un radical alkyle, alcoxy ou alkylthio contenant de 1 à 12 atomes de carbone, un radical alcoxycarbonyle ou dialkylamino contenant de 2 à 6 atomes de carbone, ou un radical phénoxy, phénylthio ou phénoxycarbonyle éventuellement porteur d'un halogène, d'un alkyle en $C_1$-$C_6$ ou d'un alcoxy en $C_1$-$C_6$, au moins un des symboles X, Y et Z désignant un atome d'hydrogène, ou

A et B représentent chacun un radical biphénylyle ou naphtyle.

2. Composés selon la revendication 1 dans lesquels A et B représentent chacun, indépendamment l'un de l'autre, un radical répondant à la formule 3 :

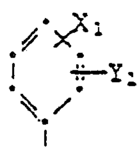

(3)

dans laquelle l'un des symboles $X_1$ et $Y_1$ représente un atome d'hydrogène, de chlore ou de brome, un radical méthyle, cyano, diméthylamino ou diéthylamino, un radical alcoxy en $C_1$-$C_{12}$, un radical alkylthio en $C_1$-$C_{12}$ ou un radical alcoxycarbonyle en $C_2$-$C_4$ et l'autre représente un atome d'hydrogène.

3. Composés selon l'une des revendications 1 et 2, dans lesquels les substituants X, Y et Z, ou $X_1$ et $Y_1$, se trouvent en méta ou en para relativement au radical de dithioxo-pyrrolo-pyrrole.

4. Procédé pour préparer les composés de formule 1 selon la revendication 1, procédé caractérisé en ce qu'on chauffe un dioxo-pyrrolo-pyrrole répondant à la formule 4 :

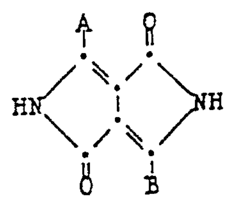

(4)

dans laquelle A et B ont les significations qui leur ont été données à la revendication 1, avec un agent de thionation.

5. Procédé selon la revendication 4 caractérisé en ce qu'on utilise, comme agent de thionation, le disulfure en 2,4 de bis-(méthoxy-4 phényl)-2,4 dithia-1,3 diphosphétanne-2,4 ou le décasulfure de tétraphosphore.

6. Procédé selon la revendication 4 caractérisé en ce qu'on effectue la thionation dans un solvant organique inerte.

7. Application des composés selon la revendication 1 comme substances photoconductrices.

8. Matière d'enregistrement électrophotographique constituée d'au moins un support conducteur, d'une couche engendrant une charge sous l'action d'un rayonnement et d'une couche transporteuse de

charge, matière caractérisée en ce qu'elle contient, dans au moins une de ces couches, au moins un composé de formule 1 selon la revendication 1.

**9.** Matière d'enregistrement électrophotographique selon la revendication 8, caractérisé en ce que la couche génératrice de charge contient un composé selon la revendication 2.

**10.** Procédé pour fabriquer une matière d'enregistrement électrophotographique, procédé caractérisé en ce qu'on applique un composé selon la revendication 1 sur un support conducteur au moyen d'un liant, ou on l'y dépose par vaporisation sous pression réduite, on traite la couche ainsi réalisée par un solvant organique à l'état liquide ou gazeux, et ensuite on dépose une seconde couche contenant un composé aromatique azoté.

**11.** Procédé de fabrication d'une matière d'enregistrement électrophotographique selon la revendication 10, procédé caractérisé en ce qu'on utilise un solvant pris dans l'ensemble constitué par l'acétone, le tétrahydrofuranne, le ciméthylformamide, le méthanol, le diméthylsulfoxyde, l'acétate c'éthyle et l'acétonitrile.